(19) Europäisches Patentamt
European Patent Office
Office européen des brevets

(11) Veröffentlichungsnummer: **0 103 544**
**A1**

(12) **EUROPÄISCHE PATENTANMELDUNG**

(21) Anmeldenummer: **83810401.6**

(22) Anmeldetag: **02.09.83**

(51) Int. Cl.³: **C 07 B 27/00**

(30) Priorität: **08.09.82 CH 5338/82**

(43) Veröffentlichungstag der Anmeldung: **21.03.84 Patentblatt 84/12**

(84) Benannte Vertragsstaaten: **CH DE FR GB IT LI**

(71) Anmelder: **CIBA-GEIGY AG, Postfach, CH-4002 Basel (CH)**

(72) Erfinder: **Spencer, Alwyn, Dr., Schützengraven 15, CH-4051 Basel (CH)**

(54) **Verfahren zur Pd-katalysierten Arylierung von Olefinen mit Arylchloriden.**

(57) Verbindungen der Formel I

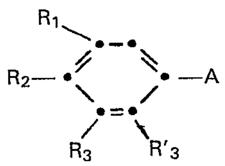

(I),

worin A $C_{2-12}$-Alkenyl, $C_{4-8}$-Cycloalkenyl oder eine Gruppierung —C(R) = C(R')—Y darstellt und Y, R, R', $R_1$, $R_2$, $R_3$ und $R'_3$ die im Patentanspruch 1 angegebene Bedeutung haben, können auf einfache und wirtschaftliche Weise dadurch hergestellt werden, dass man entsprechende Chlorbenzole in Gegenwart einer Base und katalytischer Mengen einer gegebenenfalls arsen- oder phosphorhaltigen Palladiumverbindung bei einer Temperatur von mindestens 140°C mit einer Verbindung HA umsetzt. Die Verbindungen (I) sind grösstenteils bekannt und können z.B. direkt als optische Aufheller oder als Zwischenprodukte für optische Aufheller oder Scintillatoren eingesetzt werden.

EP 0 103 544 A1

CIBA-GEIGY AG
Basel (Schweiz)

6-14087/=

# Verfahren zur Pd-katalysierten Arylierung von Olefinen mit Arylchloriden

Die Erfindung betrifft ein neues Verfahren zur Pd-katalysierten Arylierung von Olefinen mit Arylchloriden.

Gemäss U.S. Patentschrift 3.922.299 können Olefine in Gegenwart bestimmter Organometallverbindungen mit Arylhalogeniden aryliert werden. Dabei sind die Arylierungen, bezogen auf die Organometallverbindung, teils stöchiometrischer teils katalytischer Natur. Während für Arylbromide und -jodide katalytische Arylierungen beschrieben sind, werden für Arylchloride nur stöchiometrische Umsetzungen spezifisch erwähnt. Auch anderen Literaturstellen ist zu entnehmen, dass sich Olefine mit Chloraromaten nicht mittels homogener Katalyse arylieren lassen [vgl. z.B. Pure + Appl. Chem., 50, 691 (1978), Accounts of Chem. Res., 12, 146 (1979) und J. Am. Chem. Soc., 96, 1133 (1974).

In Bull. Soc. Chim. France, 2790 und 2791 (1973) wird eine heterogen katalysierte Arylierung von Styrolen mit Chloraromaten beschrieben. Andere Olefine lassen sich nach diesem Verfahren nicht oder nur schwer arylieren.

Gegenstand der Erfindung ist ein neues Verfahren zur homogenen Pd-katalysierten Arylierung von Olefinen mit Arylchloriden. Nach dem erfindungsgemässen Verfahren können Verbindungen der Formel I

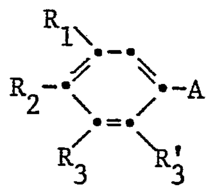

(I),

worin A $C_{2-12}$-Alkenyl, $C_{4-8}$-Cycloalkenyl oder eine Gruppe 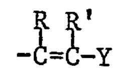 darstellt,

Y -CN, -COR", -COOH, -COOR", -CON(R")$_2$ oder 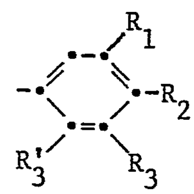 ,

R Wasserstoff, Methyl, -CN oder -COOR",

R' Wasserstoff, Methyl oder $-CH_2COOR"$,

R" $C_{1-12}$-Alkyl oder Phenyl und

$R_1$ und $R_3$ unabhängig voneinander Wasserstoff, Phenyl, $C_{1-8}$-Alkyl, $C_{1-5}$-Alkoxy, $-CH(OCH_3)_2$, $-CH(OC_2H_5)_2$,

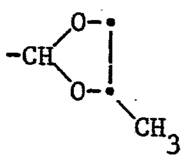 , 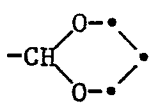 , 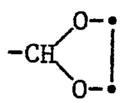 , -OH, Fluor, Chlor, $-NO_2$, -CN,

-CHO, -CO-$C_{1-4}$-Alkyl, -CO-Phenyl, -COO-$C_{1-4}$-Alkyl, -NHCO-$C_{1-4}$-Alkyl, $-CF_3$, $-NH_2$, -NH-$C_{1-4}$-Alkyl, -N($C_{1-4}$-Alkyl)$_2$, $-SO_3C_6H_5$ oder $-SO_3^{\ominus}M^{\oplus}$ bedeuten,

$R_2$ dieselbe Bedeutung wie $R_1$ bzw. $R_3$ haben kann oder eine Gruppe -CH=N-Phenyl oder $-C(R_4)=C(R_5)(R_6)$ darstellt und

$R_3'$ Wasserstoff oder Methyl ist oder $R_1$ und $R_2$ Wasserstoff bedeuten und $R_3$ und $R_3'$ zusammen -CH=CH-CH=CH- darstellen,

$R_4$ Wasserstoff, $C_{1-4}$-Alkyl, -CN oder -COO-$C_{1-4}$-Alkyl,

$R_5$ Wasserstoff, $C_{1-4}$-Alkyl, -NHCHO, $-(CH_2)_m$-COO-$C_{1-4}$-Alkyl oder $-(CH_2)_m$-CN mit m = 1 bis 4,

$R_6$ -CN oder -COO-$C_{1-4}$-Alkyl und

$M^{\oplus}$ ein Metallkation, besonders ein Alkalimetallkation, wie $K^{\oplus}$ oder vor allem $Na^{\oplus}$ bedeuten, auf besonders einfache Weise und unter Verwendung von billigen, leicht zugänglichen Ausgangsmaterilaien dadurch hergestellt werden, dass man eine Verbindung der Formel II

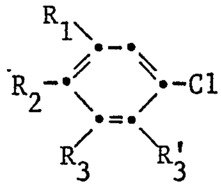

(II)

in Gegenwart einer Base und katalytischer Mengen einer gegebenenfalls arsen- oder phosphorhaltigen Palladiumverbindung bei einer Temperatur von mindestens 140°C mit einer Verbindung der Formel III

HA (III)

umsetzt, wobei A, $R_1$, $R_2$, $R_3$ und $R_3'$ die unter Formel I angegebene Bedeutung haben.

Stellt A in Formel III Alkenyl mit 3-12 C-Atomen oder $C_{4-8}$-Cycloalkenyl dar, so kann bei der Reaktion eine Verschiebung der Doppelbindung eintreten, und es entstehen im allgemeinen Isomerengemische.

Bevorzugt stellt mindestens eines von R und R' Wasserstoff dar.

Alkylgruppen R" und $R_1$ bis $R_5$, Alkoxygruppen $R_1$ und $R_3$ sowie Alkylgruppen in Substituenten $R_1$ bis $R_6$ können geradkettig oder verzweigt sein. Als Beispiele von definitionsgemässen Gruppen R" und $R_1$ bis $R_3$ seien erwähnt: Methyl, Aethyl, n-Propyl, Isopropyl, n-Butyl, sek.-Butyl, n-Pentyl, 2-Pentyl, n-Hexyl, n-Heptyl, n-Octyl, n-Decyl; Methoxy, Aethoxy, n-Propoxy, Isopropoxy, n-Butoxy, n-Pentyloxy; die Carbonsäuremethyl-, -äthyl-, -n-propyl-, -isopropyl-, -n-butyl- und sek.-butylgruppe; die Acetamid-, Propionamid-, Butyramid- und Valeramidgruppe; die N-Methylamino-, N-Aethylamino-, N-n-Propylamino- und N-n-Butylaminogruppe; die N,N-Dimethylamino-, N,N-Diäthylamino-, N,N-Di-n-propylamino-, N,N-Di-n-butylamino-, N-Methyl-N-äthylamino-, N-Methyl-N-n-propylamino- und N-Aethyl-N-n-butylaminogruppe; $-SO_3^{\ominus}Na^{\oplus}$. Als Gruppen $-C(R_4)=C(R_5)(R_6)$ kommen bevorzugt solche in Betracht, worin mindestens eines von $R_4$ und $R_5$ Wasserstoff ist, z.B.

$-CH{=}C(COOCH_3)CH_2COOCH_3$, $-CH{=}C(CH_3)COOCH_3$, $-CH{=}C(CH_3)COOC_2H_5$, $-C(COOCH_3){=}CHCOOCH_3$, $-CH{=}C(CN)CH_2CH_2CN$, $-CH{=}CHCOOC_2H_5$ und $-CH{=}CH{-}CN$.

Stellen $R_1$ bis $R_3$ Chlor dar, so können auch diese Chloratome umgesetzt werden, wobei die Reaktion selektiv verläuft. Im allgemeinen wird jedoch nur ein Chloratom umgesetzt.

Alkylgruppen R" weisen bevorzugt 1-8 und insbesondere 1-4 C-Atome auf. Besonders bevorzugt bedeutet R" Methyl, Aethyl oder n-Propyl.

Alkylgruppen $R_1$ bis $R_3$ sind bevorzugt geradkettig und weisen 1-4, besonders 1 oder 2 C-Atome auf. Bevorzugte Alkoxygruppen $R_1$ bis $R_3$ sind die Methoxy- und Aethoxygruppe. Als Alkylgruppen $R_4$ und $R_5$ und Alkylsubstituenten in Gruppen $R_1$ bis $R_6$ Methyl und Aethyl bevorzugt.

Stellt Y eine Gruppe 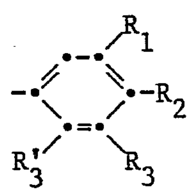 dar, so können deren Substituenten $R_1$, $R_2$, $R_3$ und $R_3'$ dieselben wie die entsprechenden Substituenten an der Verbindung der Formel II oder davon verschieden sein, wobei symmetrische oder asymmetrische Stilbenderivate entstehen. Stilbenderivate können auch dadurch hergestellt werden, dass man als Verbindung der Formel III Aethylen mit gleichen der verschiedenen Verbindungen der Formel II umsetzt.

Besonders bevorzugt verwendet man Verbindungen der Formel II, worin $R_3'$ Wasserstoff, $R_1$ und $R_3$ unabhängig voneinander Wasserstoff, Phenyl, $C_{1-3}$-Alkyl, besonders Methyl, Methoxy, $-NO_2$, -CN, -CHO, Cl, $-SO_3C_6H_5$, $-NHCO-C_{1-2}$-Alkyl, $-COO-C_{1-2}$-Alkyl, $-CO-C_{1-2}$-Alkyl, -CO-Phenyl, $-N(C_{1-2}\text{-Alkyl})_2$ bedeuten und $R_2$ dieselbe Bedeutung wie $R_1$ bzw. $R_3$

haben kann oder eine Gruppe $-C(R_4)=C(R_5)(R_6)$ darstellt, worin $R_4$ Wasserstoff, $-COOCH_3$ oder $-COOC_2H_5$, $R_5$ Wasserstoff, Methyl, Aethyl, $-CH_2COOCH_3$, $-CH_2COOC_2H_5$ oder $-CH_2CH_2CN$ und $R_6$ -CN, $-COOCH_3$ oder $-COOC_2H_5$ bedeuten, wobei bevorzugt eines von $R_4$ und $R_5$ Wasserstoff ist.

Besonders bevorzugt verwendet man Verbindungen der Formel II, worin $R_1$, $R_3$ und $R_3'$ Wasserstoff und $R_2$ Wasserstoff, Phenyl, Methyl, Methoxy, -CN, $-NO_2$, $-COOC_{1-2}$-Alkyl, -CHO, -Cl, $-COCH_3$ oder eine Gruppe $-CH{=}CH-R_6$ und $R_6$ -CN, $-COOCH_3$ oder $-COOC_2H_5$ darstellen.

Als Verbindungen der Formel III verwendet man vorzugsweise solche, worin A Alkenyl mit 2-8 C-Atomen, Cyclopentenyl und vor allem Cyclohexenyl oder eine Gruppe $-\overset{R}{C}=\overset{R'}{C}-Y$ darstellt, eines von R und R' Wasserstoff und das andere Methyl oder beide Wasserstoff, und Y Phenyl, -CN, $-CON(R'')_2$ oder -COOR" mit R" = Phenyl oder $C_{1-4}$-Alkyl, besonders Methyl oder Aethyl darstellen. Besonders bevorzugt verwendet man als Verbindung der Formel III Styrol, Aethylen, Acrylnitril, Acrylsäuremethyl- oder -äthylester, N,N-Dimethyl- und N,N-Diäthylacrylamid. Ganz besonders bevorzugt ist die Herstellung von 4-Formylzimtsäurenitril, 4,4'-(Formyl)stilben und 4,4'-(β-Cyanoäthenyl)stilben.

Bei der Umsetzung mit Aethylen können Styrol- und/oder Stilbenderivate entstehen. Die erfindungsgemässe Umsetzung kann bei Normaldruck oder unter Druck z.B. einem Druck von 0,1 bis 20 bar, durchgeführt werden. Die Reaktion lässt sich hauptsächlich durch Variation des angewendeten Druckes steuern. Stilbene werden vornehmlich bei einem Druck zwischen 0,1 und 1 bar (Normaldruck) gebildet, während bei höherem Druck, zweckmässig einem Druck zwischen 5 und 15 bar, zur Hauptsache Styrolderivate entstehen.

Die Reaktionstemperaturen liegen vorzugsweise zwischen 140 und 170°C.

Als Katalysatoren können z.B. Palladiumkomplexe der in der U.S. Patentschrift 3.922.299 beschriebenen Art, vor allem Palladium(II)Komplexe, wie z.B. von [PdCl(Aryl)$L_2$] mit L = Phosphor- oder Arsen-Ligand, $PdCl_2$, $PdBr_2$, $Pd(CN)_2$, $Pd(NO_3)_2$, $Pd(CH_3COCHCOCH_3)_2$, Pd(OOC-$C_{1-12}$-Alkyl$)_2$, besonders Palladiumacetat, oder auch Palladium(0)Komplexe, wie z.B. Komplexe von Bis-(Dibenzylidenaceton)palladium(0) und Bis-(Phenylisonitril)palladium(0), mit 3-wertigen Phosphor- oder Arsenverbindungen, wie Trialkyl-, Triaryl-, Trialkoxy- und Triphenoxyphosphinen oder -arsinen oder gemischt substituierten dreiwertigen Phosphor- oder Arsenverbindungen verwendet werden.

Als Triarylphospine oder -arsine werden solche bevorzugt, die in den Arylgruppen keine ortho-Substituenten aufweisen. Als Beispiele geeigneter Phosphor- oder Arsenverbindungen seien genannt: Triphenylarsin, Diphenylmethylphosphin, t-Butyldiphenylphosphin, Trimethylphosphin, Triäthylphosphin, Tri-n-butylphosphin, Triphenylphosphin, Phenyl-di-n-butoxyphosphin, Tri-p-tolylphosphin, Tri-(p-Methoxy- oder p-Fluorphenyl)-phosphin, Triphenylphosphit, 1,2-Bis-(diphenylphosphin)äthan, 1,3-Bis-(diphenylphosphin)propan, 1,4-Bis-(diphenylphosphin)butan und 1,2-Bis-(diphenylphosphin)benzol. Die genannten Komplexe können als solche eingesetzt oder in situ, d.h. im Reaktionsmedium, gebildet werden.

Der Phosphor- bzw. Arsenligand wird zweckmässig in 2-10-fachem molarem Ueberschuss, bezogen auf das Palladium, verwendet. Bevorzugt verwendet man als Katalysatoren Gemische aus $PdCl_2$, Palladiumacetat oder Bis-(Dibenzylidenaceton)palladium(0) und Tri-n-butylphosphin, Triphenylphosphin, Tri-p-tolylphosphin oder Triphenylphosphit, oder Komplexe aus (Chlor/Arlyl)Pd(II) mit Triphenylphosphin, z.B. Chlor-(4-formylphenyl)-bis(triphenylphosphin)Palladium(II). Besonders bevorzugt sind Gemische aus Palladiumacetat und Triphenylphosphin oder Tri-p-tolylphosphin.

Die Katalysatoren werden im allgemeinen in einer Menge von 0,01 bis 10 Mol.%, bevorzugt 0,5 bis 1 Mol.%, bezogen auf die Verbindung der Formel II, eingesetzt. Gewünschtenfalls können auch Co-Katalysatoren, wie Azoisobutyronitril, in 0,1 bis 5-fachem Ueberschuss, bevorzugt 0,5 bis 2-fachem Ueberschuss, bezogen auf die Palladiumverbindung, verwendet werden.

Die Ausgangsprodukte der Formeln II und III sind bekannt oder können nach bekannten Methoden hergestellt werden.

Als Basen können im erfindungsgemässen Verfahren sowohl anorganische als auch organische Verbindungen, die im Reaktionsmedium ausreichend löslich sind, verwendet werden. Geeignete Basen sind beispielsweise Verbindungen der Formeln IV bis VI

$(Z')^{n\oplus}\,(^{\ominus}OOCQ')_n$ , 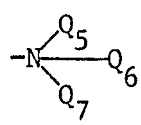 oder 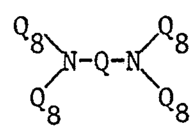

(IV) (V) (VI)

sowie cyclische tertiäre Amine, z.B. N-Methyl- oder N-Aethylpiperidin, 1,2,2,6,6-Pentamethylpiperidin, 4-Oxo-1,2,2,6,6-pentamethylpiperidin, 1,4-Diazabicyclo[2.2.2]octan (DABCO), N-Alkylmorpholine und N-Alkylpyrrolidine, wie N-Methyl- und N-Aethylmorpholine, N-Methyl- und N-Aethylpyrrolidin, oder N,N'-Dialkylpiperazine, wie N,N'-Dimethylpiperazin.

In den obigen Formeln bedeuten:

n die Zahl 1 oder 2,

Q' Phenyl oder $C_{1-17}$-Alkyl,

Z' ein Alkalimetallkation, ein Erdalkalimetallkation oder

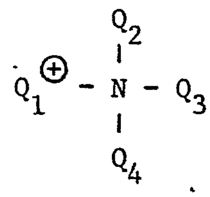 ,

Q geradkettiges oder verzweigtes Alkylen mit 2-6 C-Atomen,
$Q_1$ Wasserstoff, $C_{1-12}$-Alkyl, Cyclopentyl, Cyclohexyl, Benzyl oder Phenyl,
$Q_2$, $Q_3$ und $Q_4$ gleiches oder verschiedenes $C_{1-12}$-Alkyl,
$Q_5$ $C_{1-12}$-Alkyl, Cyclopentyl, Cyclohexyl, Phenyl oder Benzyl, das auch substituiert sein kann, beispielsweise durch ein Halogenatom, wie Chlor oder Fluor,eine Alkyl- oder Alkoxygruppe mit je 1-4 und besonders 1 oder 2 C-Atomen,
$Q_6$ und $Q_7$ gleiches oder verschiedenes $C_{1-12}$-Alkyl und
$Q_8$ Methyl oder Aethyl.

Z' in der Bedeutung eines Alkalimetallkations ist besonders das Lithium- und vor allem das Natriumkation. Alkylgruppen Q' und $Q_1$ bis $Q_7$ können geradkettig oder verzweigt sein. Stellen $Q_5$ und $Q_7$ Alkylgruppen dar, so weisen diese mit Vorteil zusammen mindestens 9 C-Atome auf, während Alkylgruppen $Q_1$ bis $Q_4$ bevorzugt je 1-4 C-Atome aufweisen. Beispiele von Verbindungen der Formeln IV bis VI sind: das Natriumacetat, -butyrat und -stearat, das Barium- und Calciumacetat, das Kalium-, Calcium- und Natriumstearat, das Lithium- und Natriumbenzoat, sowie die entsprechenden Trimethyl-, Tetramethyl-, Tetraäthyl- und Tetra-n-butylammoniumsalze; Triäthylamin, Tri-n-butylamin, Tri-(2-äthylhexylamin), Tri-n-octylamin und Tri-n-dodecylamin; N-Benzyldialkylamine, wie N-Benzyldimethylamin, N-Benzyldiäthylamin, N-(4-Chlorbenzyl)-dimethylamin und N-(3-Methyl- oder 3-Methoxybenzyl)-dimethylamin; N,N,N',N'-Tetramethyl- und N,N,N',N'-Tetraäthyl-äthylendiamin, N,N,N',N'-Tetramethyl-1,3-diaminopropan und N,N,N',N'-Tetramethyl-1,6-diaminohexan.

Bevorzugt verwendet man als Basen Carboxylate der Formel IV oder tertiäre Amine der vorerwähnten Art, vor allem N-Aethylmorpholin oder Verbindungen der Formel V, worin $Q_5$ 4-Chlorbenzyl, 4-Methylbenzyl oder 4-Methoxybenzyl und insbesondere Benzyl und $Q_6$ und $Q_7$ je Alkyl mit 1-4 C-Atomen, vor allem 1 oder 2 C-Atomen, darstellen, oder worin $Q_5$, $Q_6$ und $Q_7$ je Alkyl mit 3-12 C-Atomen darstellen. Besonders bevorzugt sind Natriumacetat, Natriumbenzoat, N-Benzyldimethylamin, N-Aethylmorpholin und Tri-n-butylamin.

Sind die Verbindungen der Formeln II oder III flüssig, so kann die Umsetzung ohne Zusatz eines Lösungsmittels durchgeführt werden. Bevorzugt wird die Reaktion jedoch in einem gegenüber den Reaktionspartnern inerten organischen Lösungsmittel durchgeführt. Geeignete inerte organische Lösungsmittel sind je nach Reaktionskomponenten z.B. gegebenenfalls chlorierte aliphatische, cycloaliphatische oder aromatische Kohlenwasserstoffe, wie n-Pentan, n-Heptan, n-Octan, Cyclopentan, Cyclohexan, Benzol, Toluol, Xylole und Chlorbenzol; aromatische, aliphatische und cyclische Aether, wie Anisol, Diäthyläther, Di-isopropyläther, Tetrahydrofuran und Dioxan; N-substituierte Morpholine, wie N-Methyl- und N-Formylmorpholin; Nitrile, besonders Benzonitril und Alkylnitrile mit 2 bis 5 C-Atomen, wie Propionitril und Butyronitril; 3-Methoxypropionitril und 3-Aethoxypropionitril; Dialkylsulfoxide, wie Dimethyl- und Diäthylsulfoxid; N,N-Dialkylamide von aliphatischen Monocarbonsäuren mit 1-3 C-Atomen im Säureteil, wie N,N-Dimethylformamid und N,N-Dimethylacetamid; Hexamethylphosphorsäuretriamid; Alkohole mit bis zu 8 C-Atomen, wie Aethanol, n-Propanol und tert.-Butanol; aliphatische und cyclische Ketone, wie Aceton, Diäthylketon, Methylisopropylketon, Cyclopentanon, Cyclohexanon, 1,3-Dimethyl-2-imidazolidinon und 1,3-Dimethyl-3,4,5,6-tetrahydro-2-(1H)-pyrimidinon; Tetramethylharnstoff; Ester, wie Ester der Kohlensäure, z.B. Diäthylcarbonat, Nitromethan; Alkyl- oder Alkoxyalkylester von aliphatischen Monocarbonsäuren mit insgesamt 2-8 C-Atomen, wie Essigsäuremethyl-, -äthyl-, -n-butyl- und -isobutylester, Buttersäureäthyl- und -n-butylester sowie 1-Acetoxy-2-äthoxyäthan und

1-Acetoxy-2-methoxyäthan oder Triäthylphosphat. Bevorzugte Lösungsmittel sind N,N-Dialkylamide von aliphatischen Monocarbonsäuren oder oben erwähnten Art, besonders N,N-Dimethylformamid, und Hexamethylphosphorsäuretriamid. In einer bevorzugten Ausführungsform werden polare Lösungsmittel zusammen mit einem Carbonsäuresalz eingesetzt.

Die erfindungsgemäss herstellbaren Verbindungen und deren Anwendung sind zum grossen Teil bekannt. Sie können beispielsweise direkt als optische Aufheller oder aber als Zwischenprodukte für optische Aufheller oder Scintillatoren eingesetzt werden. Derartige optische Aufheller bzw. Scintillatoren sind z.B. in der U.S. Patentschrift 4.108.887 und der britischen Patentschrift 2.015.021 beschrieben. Die erfindungsgemäss hergestellten Verbindungen können ferner in an sich bekannter Weise, gegebenenfalls unter Einführung von geeigneten funktionellen Gruppen, wie Aminogruppen, und/oder durch Sulfonierung der aromatischen Reste in Farbstoff oder optische Aufheller übergeführt werden [vgl. z.B. Encyclopedia of Chemical Technology, 2. Auflage Bd. 19, S. 1 bis 16]. Stilbene und Stilbenderivate finden auch Anwendung als Klebstoffadditive, Insektizide oder Lichtstabilisatoren; vgl. z.B. Chemical Abstracts 78, 39352; 84, 137386 und 85, 22416. Styrole und Styrolderivate eignen sich auch zur Herstellung von Homo- oder Copolymeren.

In den folgenden Beispielen wurden die Umsetzungen, sofern nichts anderes angegeben ist, im geschlossenen Druckrohr durchgeführt.

Beispiel 1: Unter Argon werden zu 10 ml N,N-Dimethylformamid (DMF) in einem Druckrohr 1,41 g (10 mMol) 4-Chlorbenzaldehyd, 1,33 ml (20 mMol) Acrylnitril, 1,64 g (20 mMol) wasserfreies Natriumacetat, 0,077 g (0,1 mMol) Chlor-(4-formylphenyl)-bis-(triphenylphosphin) Palladium(II), und 0,0525 g (0,2 mMol) Triphenylphosphin zugegeben. Das Rohr wird verschlossen, und das Gemisch wird während 6 Stunden bei 150°C gerührt. Nach dem Filtrieren und Abdestillieren des DMF am Rotationsverdampfer wird das Rohprodukt im Vakuum destilliert.

Man erhält 0,29 g (1,8 mMol) 4-Formylzimtsäurenitril als fast weissen Feststoff; Ausbeute 18% d.Th. Es ist ein Isomerengemisch bestehend aus ca. 68% trans- und ca. 32% cis-Isomer. Nach dem Umkristallisieren aus n-Hexan/Toluol wird das reine trans-Isomere in Form schwach gelber Blättchen erhalten; Fp. 121-2°C. Analyse für $C_{10}H_7NO$ (Molgewicht 157,17): berechnet C 76,42, H 4,49, N 8,91, O 10,18, gefunden C 75,91, H 4,50, N 9,1, O 10,30.

Beispiel 2: Es wird wie in Beispiel 1 beschrieben vorgegangen, jedoch bei einer Reaktionstemperatur von 170°C statt 150°C. Man erhält 0,22 g (1,4 mMol) 4-Formylzimtsäurenitril, entsprechend einer Ausbeute von 14% d.Th. (trans 55%, cis 45%).

Beispiel 3: Es wird wie in Beispiel 1 beschrieben vorgegangen, jedoch unter Verwendung von 0,83 ml (12,5 mMol) Acrylnitril und 1,03 g (12,5 mMol) Natriumacetat. Man erhält 0,39 g (2,5 mMol) 4-Formylzimtsäurenitril, entsprechend einer Ausbeute von 24% d.Th. (trans 69%, cis 31%).

Beispiel 4: Es wird wie in Beispiel 3 beschrieben vorgegangen, jedoch unter Verwendung von 0,0224 g (0,1 mMol) Palladium(II)acetat und 0,1048 g (0,4 mMol) Triphenylphosphin. Man erhält 0,36 g (2,3 mMol) 4-Formylzimtsäurenitril, entsprechend einer Ausbeute von 23% d.Th. (trans 69%, cis 31%).

Beispiel 5: Es wird wie in Beispiel 3 beschrieben vorgegangen, jedoch unter Verwendung von 0,67 ml (10 mMol) Acrylnitril und 0,82 g (10 mMol) Natriumacetat. Man erhält 0,44 g (2,8 mMol) 4-Formylzimtsäurenitril, entsprechend einer Ausbeute von 28% d.Th. (trans 70%, cis 30%).

Beispiele 6-10: Es wird wie in Beispiel 5 beschrieben vorgegangen, jedoch unter Verwendung von jeweils 0,4 mMol der folgenden Phosphorliganden anstelle von Triphenylphosphin:

| Beispiel Nr. | Phosphorligand | 4-Formylzimtsäurenitril | | |
|---|---|---|---|---|
| | | (mMol) | Ausbeute % d.Th. | trans/cis |
| 6 | t-Butyldiphenylphosphin | 1,2 | 12 | 78/22 |
| 7 | Tris-(p-methoxyphenyl)-phosphin | 0,8 | 8 | 68/32 |
| 8 | Tris- p -tolylphosphin | 3,4 | 34 | 72/28 |
| 9 | Tris-o-tolylphosphin | 0,4 | 4 | 65/35 |
| 10 | Tris-(p-fluorphenyl)-phosphin | 1,7 | 17 | 63/37 |

Beispiele 11-14: Es wird wie in Beispiel 5 beschrieben vorgegangen, jedoch unter Verwendung von jeweils 0,1 mMol der folgenden Phosphorliganden:

| Beispiel Nr. | Phosphorligand | 4-Formylzimtsäurenitril | | |
|---|---|---|---|---|
| | | (mMol) | % d.Th. | trans/cis |
| 11 | 1,2-Bis(diphenylphosphin)äthan | 2,2 | 22 | 71/29 |
| 12 | 1,3-Bis-(diphenylphosphin)propan | 0,8 | 8 | 69/31 |
| 13 | 1,4-Bis-(diphenylphosphin)butan | 0,7 | 7 | 67/33 |
| 13 | 1,2-Bis-(diphenylphosphin)benzol | 0,3 | 3 | 55/45 |

Beispiel 15: Es wird wie in Beispiel 11 beschrieben vorgegangen, jeoch unter Verwendung von 0,2 mMol 1,2-Bis-(diphenylphosphin)-äthan. Man erhält 0,24 g (1,5 mMol) 4-Formylzimtsäurenitril, entsprechend einer Ausbeute von 15% d.Th. (trans 70%, cis 30%).

Beispiel 16: Es wird wie in Beispiel 5 beschrieben vorgegangen, jedoch mit einer Reaktionszeit von 3 Stunden. Man erhält 0,34 g (2,2 mMol) 4-Formylzimtsäurenitril, entsprechend einer Ausbeute von 22% d.Th. (trans 68%, cis 32%).

Beispiel 17: Es wird wie in Beispiel 5 beschrieben vorgegangen, jedoch mit einer Reaktionszeit von 16 Stunden. Man erhält 0,26 g (1,7 mMol) 4-Formylzimtsäurenitril, entsprechend einer Ausbeute von 17% d.Th. (trans 74%, cis 26%).

Beispiel 18: Es wird wie in Beispiel 5 beschrieben vorgegangen, jedoch bei einer Temperatur von 160°C. Man erhält 0,24 g (1,5 mMol) 4-Formylzimtsäurenitril, entsprechend einer Ausbeute von 15% d.Th. (trans 57%, cis 43%).

Beispiele 19-21: Es wird wie in Beispiel 5 beschrieben vorgegangen, jedoch mit verschiedenen Mengen Triphenylphosphin und Palladiumacetat anstelle der dort verwendeten Katalysatoren/Liganden:

| Beispiel Nr. | Mol-Verhältnis Triphenylphosphin/Palladium(II)acetat | 4-Formylzimtsäurenitril mMol | % d.Th. | trans/cis |
|---|---|---|---|---|
| 19 | 2 | 0,3 | 3 | 61/39 |
| 20 | 6 | 2,6 | 26 | 69/31 |
| 21 | 8 | 1,2 | 12 | 64/36 |

Beispiele 22-24: Es wird wie in Beispiel 5 beschrieben vorgegangen, jedoch unter Verwendung von jeweils 10 mMol der folgenden Basen:

| Beispiel Nr. | Base | 4-Formylzimtsäurenitril mMol | % d.Th. | trans/cis |
|---|---|---|---|---|
| 22 | Tri-ni-butylamin | 1,1 | 11 | 67/33 |
| 23 | N-Benzyldimethylamin | 2,0 | 20 | 69/31 |
| 24 | N-Aethylmorpholin | 1,9 | 19 | 74/26 |

Beispiele 25-30: Es wird wie in Beispiel 5 beschrieben vorgegangen, jedoch unter Verwendung der folgenden Lösungsmittel anstelle von DMF:

| Beispiel Nr. | Lösungsmittel | 4-Formylzimtsäurenitril mMol | % d.Th. | trans/cis |
|---|---|---|---|---|
| 25 | N,N-Dimethylacetamid | 2,5 | 25 | 65/35 |
| 26 | N-Formylmorpholin | 2,8 | 28 | 79/21 |
| 27 | N-Methylmorpholin | 2,9 | 29 | 70/30 |
| 28 | Dimethylsulfoxid | 0,3 | 3 | 75/25 |
| 29 | Hexamethylphosphorsäure-triamid | 4,9 | 49 | 75/25 |
| 30 | Aethylenglykol | 0,3 | 3 | 58/42 |

Beispiel 31: Es wird wie in Beispiel 5 beschrieben vorgegangen, jedoch unter Verwendung von 10 mMol Triäthylamin und 10 ml Propionitril anstelle von Natriumacetat und DMF. Man erhält 0,15 mMol 4-Formylzimtsäurenitril, entsprechend einer Ausbeute von 15% d.Th. (trans 58%, cis 42%).

Beispiel 32: Es wird wie in Beispiel 5 beschrieben vorgegangen, jedoch unter Verwendung von 0,1 mMol Azobisisobutyronitril als Co-Katalysator. Man erhält das 4-Formylzimtsäurenitril in einer Ausbeute von 59% d.Th. (5,9 mMol; trans 82%, cis 18%).

Beispiele 33-38: Unter Argon werden zu 20 ml DMF in einem Druckrohr 50 mMol der unten angegebenen Chloraromaten, 5,42 ml (50 mMol) Acrylsäureäthylester, 4,1 g (50 mMol) wasserfreies Natriumacetat, 0,1122 g (0,5 mMol) Palladium(II)acetat und 0,524 g (2 mMol) Triphenylphosphin zugegeben. Das Rohr wird verschlossen, und das Gemisch wird während 6 Stunden bei 150°C gerührt. Nach der Aufarbeitung gemäss Beispiel 1 wird das Produkt entweder destilliert oder umkristallisiert.

| Bsp. Nr. | Chloraromat | Produkt (mMol) | Ausbeute % d.Th. |
|---|---|---|---|
| 33 | 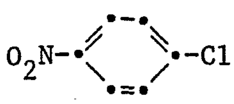 | 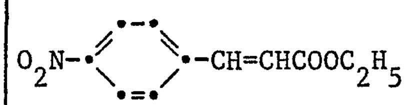 (10,5) | 21 |
| 34 | 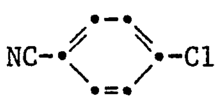 | 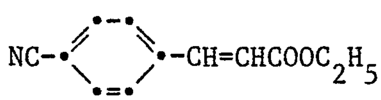 (16) | 32 |
| 35 | 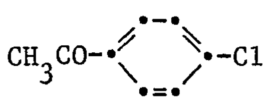 | 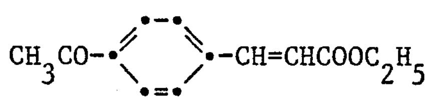 (13) | 26 |
| 36 | 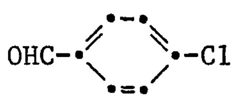 | 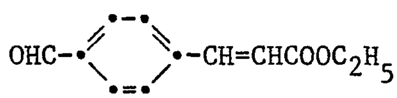 (19,5) | 39 |
| 37 | 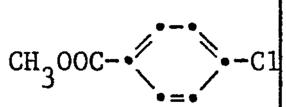 | 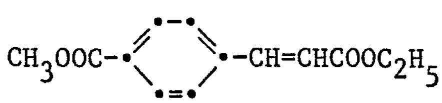 (25,5) | 51 |
| 38 | 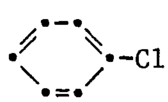 | 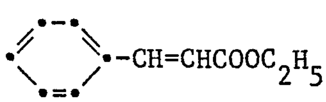 (2,0) | 4 |

Beispiele 39-41: Es wird wie in Beispiel 36 beschrieben vorgegangen, jedoch unter Verwendung von jeweils 50 mMol der folgenden Olefine:

| Beispiel Nr. | Olefin | Produkt (mMol) | Ausbeute % d.Th. |
|---|---|---|---|
| 39 | 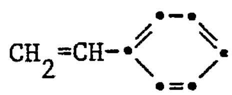 | 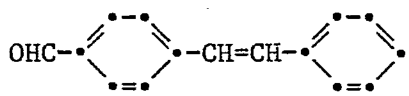 (18,5) | 37 |
| 40 | $CH_2=CHCON(C_2H_5)_2$ | 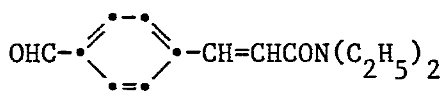 (22,5) | 45 |
| 41 | $CH_2=CHCN$ | 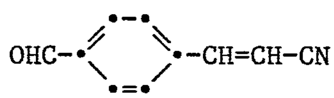 (11,5) | 23 |

Beispiel 42: Unter Argon werden zu 20 ml Hexamethylphosphorsäuretriamid in einem Druckrohr 10,83 g (50 ml) 4-Chlorbenzophenon, 5,0 g (50 mMol) Acrylsäureäthylester, 4,1 g (50 mMol) wasserfreies Natriumacetat, 0,1122 g (0,5 mMol) Palladium(II)acetat und 0,524 g (2,0 mMol) Triphenylphosphin zugegeben. Das Rohr wird verschlossen, und das Gemisch wird während 6 Stunden bei 150°C gerührt. Das Rohprodukt wird über Kieselgel filtriert und anschliessend aus n-Hexan umkristallisiert. Man erhält 6,13 g (21,9 mMol) 4-(2-Aethoxycarbonylvinyl)-benzophenon als weisse Kristalle vom Smp. 82°C, entsprechend einer Ausbeute von 44% d.Th. (trans-Isomeres). Analyse für $C_{18}H_{16}O_3$ (Molgewicht 280,32): berechnet C 77,13, H 5,76, O 17,12, gefunden C 77,20, H 5,68, O 17,35.

Beispiel 43: Es wird wie in Beispiel 42 beschrieben vorgegangen, jedoch unter Verwendung von 8,53 g (50 mMol) 4-Chlorbenzoesäuremethylester und 5,21 g (50 mMol) Styrol anstelle von 4-Chlorbenzophenon und Acrylsäureäthylester. Das Rohprodukt wird abfiltriert und aus einem Methanol/Aceton-Gemisch umkristallisiert. Man erhält 5,26 g (22 mMol) Stilben-4-carbonsäuremethylester als weisse Kristalle vom Smp. 160°C, entsprechend einer Ausbeute von 44% d.Th. Analyse für $C_{16}H_{14}O_2$ (Molgewicht 238,29): berechnet C 80,66, H 5,92, O 13,43, gefunden C 80,53 H 5,86, O 13,63.

Beispiel 44: Es wird wie in Beispiel 42 beschrieben vorgegangen, jedoch unter Verwendung von 7,73 g (50 mMol) 4-Chloracetophenon und 6,35 g (50 ml) N,N-Diäthylacrylamid anstelle von 4-Chlorbenzophenon und Acrylsäureäthylester. Das Rohprodukt wird aus Cyclohexan umkristallisiert. Man erhält 7,43 g (30,3 mMol) N,N-Diäthyl-4-acetylzimtsäureamid als weisse Kristalle vom Smp. 70°C, entsprechend einer Ausbeute von 61% d.Th. Analyse für $C_{15}H_{19}NO_2$ (Molgewicht 245,2): berechnet C 73,40, H 7,83, N 5,71, O 13,05, gefunden C 73,58, H 7,91, N 5,72, O 13,07.

Beispiele 45-46: Es wird wie in Beispiel 44 beschrieben vorgegangen, jedoch unter Verwendung der folgenden Mengen Palladium(II)acetat und Triphenylphosphin:

| Beispiel Nr. | Palladium(II)-acetat (mMol) | Triphenylphos-phin (mMol) | Produkt (mMol) | Ausbeute % d.Th. |
|---|---|---|---|---|
| 45 | 0,25 | 1 | 28 | 56 |
| 46 | 0,05 | 0,2 | 6 | 12 |

Beispiel 47: Es wird wie in Beispiel 43 beschrieben vorgegangen, jedoch unter Verwendung von 7,88 g (50 mMol) 4-Chlornitrobenzol und 20 ml N,N-Dimethylformamid anstelle von 4-Chlorbenzoesäuremethylester und Hexamethylphosphorsäuretriamid. Das Rohprodukt wird abfiltriert und aus Methanol umkristallisiert. Man erhält 2,32 g (10,3 mMol) 4-Nitrostilben als gelbe Kristalle vom Smp. 155°C, entsprechend einer

Ausbeute von 21% d.Th. Analyse für $C_{14}H_{11}NO_2$ (Molgewicht 225,25): berechnet C 74,65, H 4,92, N 6,22, gefunden C 74,84, H 5,01, N 6,02.

Beispiel 48: Es wird wie in Beispiel 44 beschrieben vorgegangen, jedoch unter Verwendung von 6,33 g (50 mMol) 4-Chlortoluol anstelle von 4-Chloracetophenon. Man erhält 5,73 g (26,4 mMol) N,N-Diäthyl-4-methylzimtsäureamid als gelbe Kristalle vom Sdp. 160°C/0,26 mbar, entsprechend einer Ausbeute von 53% d.Th. Analyse für $C_{14}H_{19}NO$ (Molgewicht 205,30): berechnet C 77,35, H 8,84, N 6,45, gefunden C 76,16, H 9,13, N 6,52 (trans-Isomeres).

Beispiel 49: Es wird wie in Beispiel 33 beschrieben vorgegangen, jedoch unter Verwendung von 20 ml Hexamethylphosphorsäuretriamid anstelle von N,N-Dimethylformamid. Man erhält 6,08 g (27,5 mMol) 4-Nitrozimtsäureäthylester, entsprechend einer Ausbeute von 55% d.Th.

Beispiel 50: Unter Argon werden zu 20 ml N,N-Dimethylformamid in einem Druckrohr 7,35 g (50 mMol) 1,4-Dichlorbenzol, 12,7 g (100 mMol) N,N-Diäthylacrylamid, 8,2 g (100 mMol) wasserfreies Natriumacetat, 0,2244 g (1 mMol) Palladium(II)acetat und 1,048 g (4 mMol) Triphenylphosphin zugegeben. Das Rohr wird verschlossen, und das Gemisch wird während 6 Stunden bei 150°C gerührt. Das Rohprodukt wird über Kieselgel filtriert und anschliessend destilliert. Man erhält 6,98 g (29,4 mMol) N,N-Diäthyl-4-chlorzimtsäureamid als gelbe Flüssigkeit; Sdp. 160°C/0,26 mbar, entsprechend einer Ausbeute von 59% d.Th.

Beispiel 51: Es wird wie in Beispiel 38 beschrieben vorgegangen, jedoch unter Verwendung von 7,91 g (50 mMol) Itaconsäuredimethylester anstelle von Acrylsäureäthylester. Man erhält 5,03 g (21,5 mMol) 4-Phenylitaconsäuredimethylester als farblose Flüssigkeit vom Sdp. 130°C/0,13 mbar, entsprechend einer Ausbeute von 43% d.Th.

Beispiel 52: Es wird wie in Beispiel 36 beschrieben vorgegangen, jedoch unter Verwendung von 5,2 g (50 mMol) Styrol anstelle von Acrylsäureäthylester. Das Rohprodukt wird aus einem n-Hexan/Cyclohexan-Gemisch umkristallisiert. Man erhält 3,81 g 4-Formylstilben als hellgelbe Kristalle vom Smp. 112-13°C, entsprechend einer Ausbeute von 37% d.Th.

Beispiel 53: Es wird wie in Beispiel 44 beschrieben vorgegangen, jedoch unter Verwendung von 6,33 g (50 mMol) 2-Chlortoluol anstelle von 4-Chloracetophenon. Das Rohprodukt wird destilliert. Man erhält 4,8 g (22 mMol) N,N-Diäthyl-2-methylzimtsäureamid als hellgelbe Flüssigkeit vom Sdp. 135°C/0,26 mbar, entsprechend einer Ausbeute von 44% d.Th.

Beispiel 54: Es wird wie in Beispiel 53 beschrieben vorgegangen, jedoch unter Verwendung von 6,33 g (50 mMol) 3-Chlortoluol anstelle von 2-Chlortoluol. Das Rohprodukt wird destilliert. Man erhält 4,35 g (20 mMol) N,N-Diäthyl-3-methylzimtsäureamid vom Sdp. 130°C/0,13 mbar, entsprechnd einer Ausbeute von 40% d.Th.

Beispiel 55: In einem Rückflussapparat werden unter Durchleitung von Aethylen bei Normaldruck 20 ml Hexamethylphosphorsäuretriamid vorgelegt und 8,18 g (50 mMol) 4-Chlorzimtsäurenitril, 7,2 g (50 mMol) Natriumbenzoat, 0,1122 g (0,5 mMol) Palladium(II)acetat und 0,524 g (2 mMol) Triphenylphosphin zugegeben. Das Gemisch wird während 6 Stunden bei 150°C unter weiterer Aethylendurchleitung gerührt. Danach wird es bei Raumtemperatur in 200 ml 2N Natronlauge eingegossen. Der entstandene Niederschlag wird abfiltriert, mit 60 ml Methanol gewaschen und in 30 ml heissem DMF gelöst. Durch Zugabe von 30 ml Wasser wird das Produkt ausgefällt. Nach dem Abfiltrieren und Trocknen erhält man 2,32 g (8,2 mMol) 4,4'-Bis-(2-cyanovinyl)-stilben als gelbe Kristalle vom Smp. 200-2°C, entsprechend einer Ausbeute von 33% d.Th. (trans, trans, trans-Isomeres).

Beispiel 56: Es wird wie in Beispiel 55 beschrieben vorgegangen, jedoch unter Verwendung von 20 ml DMF anstelle von Hexamethylphosphorsäuretriamid. Man erhält 1,4 g (5 mMol) 4,4'-Bis-(cyanovinyl)-stilben, entsprechend einer Ausbeute von 20% d.Th.

Beispiel 57: Es wird wie in Beispiel 55 beschrieben vorgegangen, jedoch unter Verwendung von 7,03 g (50 mMol) 4-Chlorbenzaldehyd anstelle von 4-Chlorzimtsäurenitril. Das Rohprodukt wird aus Toluol umkristallisiert. Man erhält 0,49 g (2,1 mMol) Stilben-4,4'-dialdehyd als gelbe Kristalle vom Smp. 167-9°C, entsprechend einer Ausbeute von 8% d.Th.

Beispiel 58: Es wird wie in Beispiel 57 beschrieben vorgegangen, jedoch unter Verwendung von 20 ml DMF anstelle von Hexamethylphosphorsäuretriamid. Man erhält 0,45 g (1,9 mMol) Stilben-4,4'-dialdehyd, entsprechend einer Ausbeute von 8% d.Th.

Beispiel 59: Es wird wie in Beispiel 55 beschrieben vorgegangen, jedoch unter Verwendung von 4,1 g (50 mMol) Natriumacetat (wasserfrei), 50 ml N-Methylpyrrolidon und 10,53 g (50 mMol) 4-Chlorzimtsäureäthylester anstelle von Natriumbenzoat, Hexamethylphosphorsäuretriamid und 4-Chlorzimtsäurenitril. Man erhält 4,88 g (13 mMol) 4,4'-(2-Aethoxycarbonylvinyl)-stilben als hellgelbe Kristalle vom Smp. 239-41°C, entsprechend einer Ausbeute von 26% d.Th.

Beispiele 60-61: Es wird wie in Beispiel 40 beschrieben vorgegangen, jedoch unter Verwendung von jeweils 20 ml der unten angegebenen Lösungsmittel:

| Beispiel Nr. | Lösungsmittel | Ausbeute an OHC– 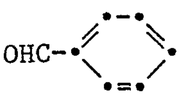 –$CH=CHCON(CH_3)_2$ % d.Th. |
|---|---|---|
| 60 | 1,3-Dimethyl-2-imidazolidinon | 28 |
| 61 | 1,3-Dimethyl-3,4,5,6-tetrahydro-2-(1H)-pyrimidinon | 46 |

Patentansprüche

1. Verfahren zur Herstellung von Verbindungen der Formel (I)

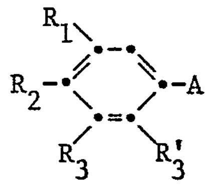 (I),

worin A $C_{2-12}$-Alkenyl, $C_{4-8}$-Cycloalkenyl oder eine Gruppe 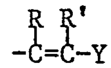 darstellt,

Y -CN, -COR", -COOH, -COOR", $-CON(R")_2$ oder 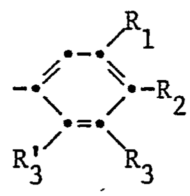 ,

R Wasserstoff, Methyl, -CN oder -COOR",

R' Wasserstoff, Methyl oder $-CH_2COOR"$,

R" $C_{1-12}$-Alkyl oder Phenyl und

$R_1$ und $R_3$ unabhängig voneinander Wasserstoff, Phenyl, $C_{1-8}$-Alkyl, $C_{1-5}$-Alkoxy, $-CH(OCH_3)_2$, $-CH(OC_2H_5)_2$,

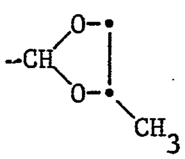 , 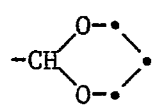 , 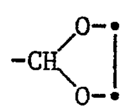 , -OH, Fluor, Chlor, $-NO_2$, -CN,

-CHO, -CO-$C_{1-4}$-Alkyl, -CO-Phenyl, -COO-$C_{1-4}$-Alkyl, -NHCO-$C_{1-4}$-Alkyl, $-CF_3$, $-NH_2$, -NH-$C_{1-4}$-Alkyl, -N($C_{1-4}$-Alkyl$)_2$, $-SO_3C_6H_5$ oder $-SO_3M^{\oplus}$ bedeuten, $R_2$ dieselbe Bedeutung wie $R_1$ bzw. $R_3$ haben kann oder eine Gruppe -CH=N-Phenyl oder $-C(R_4)=C(R_5)(R_6)$ darstellt und $R_3'$ Wasserstoff oder Methyl ist oder $R_1$ und $R_2$ Wasserstoff bedeuten und $R_3$ und $R_3'$ zusammen -CH=CH-CH=CH- darstellen,

$R_4$ Wasserstoff, $C_{1-4}$-Alkyl, -CN oder -COO-$C_{1-4}$-Alkyl,

$R_5$ Wasserstoff, $C_{1-4}$-Alkyl, -NHCHO, $-(CH_2)_m$-COO-$C_{1-4}$-Alkyl oder $-(CH_2)_m$-CN mit m = 1 bis 4,

$R_6$ -CN oder -COO-$C_{1-4}$-Alkyl und

$M^{\oplus}$ ein Metallkation bedeuten, dadurch gekennzeichnet, dass man eine Verbindung der Formel II

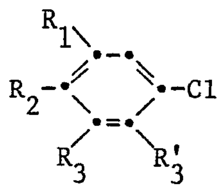

(II)

in Gegenwart einer Base und katalytischer Mengen einer gegebenenfalls arsen- oder phosphorhaltigen Palladiumverbindung bei einer Temperatur von mindestens 140°C mit einer Verbindung der Formel III

HA (III)

umsetzt, wobei A, $R_1$, $R_2$, $R_3$ und $R_3'$ die unter Formel I angegebene Bedeutung haben.

2. Verfahren nach Patentanspruch 1, dadurch gekennzeichnet, dass man eine Verbindung der Formel III verwendet, worin eine Gruppe A -C(R)=C(R')-Y darstellt und mindestens eines von R und R' Wasserstoff ist.

3. Verfahren nach Patentanspruch 1, dadurch gekennzeichnet, dass man eine Verbindung der Formel II verwendet, worin $R_3'$ Wasserstoff, $R_1$ und $R_3$ unabhängig voneinander Wasserstoff, Phenyl, $C_{1-3}$-Alkyl, Methoxy, $-NO_2$, -CN, -CHO, Cl, $-SO_3C_6H_5$, -NHCO-$C_{1-2}$-Alkyl, -COO-$C_{1-2}$-Alkyl, -CO-$C_{1-2}$-Alkyl, -CO-Phenyl oder -N($C_{1-2}$-Alkyl)$_2$ bedeuten und $R_2$ dieselbe Bedeutung wie $R_1$ bzw. $R_3$ haben kann oder eine Gruppe $-C(R_4)=C(R_5)(R_6)$ darstellt, worin $R_4$ Wasserstoff, $-COOCH_3$ oder $-COOC_2H_5$, $R_5$ Wasserstoff, Methyl, Aethyl, $-CH_2COOCH_3$, $-CH_2COOC_2H_5$ oder $-CH_2CH_2CN$ und $R_6$ -CN, $-COOCH_3$ oder $-COOC_2H_5$ bedeuten, wobei eines von $R_4$ und $R_5$ Wasserstoff ist.

4. Verfahren nach Patentanspruch 1, dadurch gekennzeichnet, dass man eine Verbindung der Formel II verwendet, worin $R_1$, $R_3$ und $R_3'$ Wasserstoff und $R_2$ Wasserstoff, Phenyl, Methyl, Methoxy, -CN, $-NO_2$, $-COOC_{1-2}$-

Alkyl, -CHO, Cl, $-COCH_3$ oder eine Gruppe $-CH=CH-R_6$ und $R_6$ -CN, $-COOCH_3$ oder $-COOC_2H_5$ darstellen.

5. Verfahren nach Patentanspruch 1, dadurch gekennzeichnet, dass man eine Verbindung der Formel III verwendet, worin A Alkenyl mit 2-8 C-Atomen, Cyclopentenyl, Cyclohexenyl oder eine Gruppe -C(R)=C(R')-Y darstellt, eines von R und R' Wasserstoff und das andere Methyl oder beide Wasserstoff und Y Phenyl, -CN, $-CON(R")_2$ oder -COOR" mit R" = Phenyl oder $C_{1-4}$-Alkyl bedeuten.

6. Verfahren nach Patentanspruch 1, dadurch gekennzeichnet, dass man als Verbindung der Formel III Styrol, Aethylen, Acrylnitril, Acrylsäuremethyl- oder -äthylester, N,N-Dimethyl- oder N,N-Diäthylacrylamid verwendet.

7. Verfahren nach Patentanspruch 1, dadurch gekennzeichnet, dass man 4-Formylzimtsäurenitril herstellt.

8. Verfahren nach Patentanspruch 1, dadurch gekennzeichnet, dass man die Umsetzung bei einer Temperatur zwischen 140 und 170°C vornimmt.

9. Verfahren nach Patentanspruch 1, dadurch gekennzeichnet, dass man als Katalysator Chlor-(4-formylphenyl)-bis-(triphenylphosphin)-Palladium(II) verwendet.

10. Verfahren nach Patentanspruch 1, dadurch gekennzeichnet, dass man als Katalysator ein Gemisch aus Palladiumacetat und Triphenylphosphin oder Tri-p-tolylphosphin verwendet.

11. Verfahren nach Patentanspruch 1, dadurch gekennzeichnet, dass man den Katalysator in einer Menge von 0,5 bis 1 Mol.%, bezogen auf die Verbindung der Formel II, einsetzt.

12. Verfahren nach Patentanspruch 1, dadurch gekennzeichnet, dass man als Base Natriumacetat, Natriumbenzoat, N-Benzyldimethylamin, N-Aethylmorpholin oder Tri-n-butylamin verwendet.

13. Verfahren nach Patentanspruch 1, dadurch gekennzeichnet, dass man als Lösungsmittel N,N-Dialkylamide von aliphatischen Monocarbonsäuren mit 1-3 C-Atomen im Säureteil, besonders N,N-Dimethylformamid, oder Hexamethylphosphorsäuretriamid verwendet.

14. Verfahren nach Patentanspruch 1, dadurch gekennzeichnet, dass man die Alkali- oder Erdalkalicarbonsäuresalze zusammen mit einem polaren Lösungsmittel einsetzt.

FO 7.3/(HO)/jt*

0103544

Europäisches Patentamt

# EUROPÄISCHER RECHERCHENBERICHT

Nummer der Anmeldung

EP 83 81 0401

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | BULLETIN DE LA SOCIETE CHIMIQUE DE FRANCE, September/Oktober 1973, Seiten 2791-2794, Paris (FR); MARC JULIA et al.: "Etude de la condensation de chlorures aromatiques avec les oléfines catalysée par le palladium". *Insgesamt* | 1-14 | C 07 B 27/00 |
| Y,D | US-A-3 922 299 (R.F.HECK) *Ansprüche 1-7* | 1-14 | |
| Y,P | EP-A-0 078 768 (CIBA-GEOGY) *Seiten 25-29* | 1-14 | |
| | | | RECHERCHIERTE SACHGEBIETE (Int. Cl. 3) |
| | | | C 07 B 27/00<br>C 07 C 121/00 |

Der vorliegende Recherchenbericht wurde für alle Patentansprüche erstellt.

| Recherchenort | Abschlußdatum der Recherche | Prüfer |
|---|---|---|
| DEN HAAG | 12-12-1983 | VERHULST W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82